# EUROPEAN PATENT APPLICATION

(11) **EP 2 218 393 A1**
(43) Date of publication of application: **18.08.2010**
(21) Application number: 10001384.6
(22) Date of filing: 11.02.2010
(51) Int. Cl.: A61B 5/00, A63F 13/00, G06F 19/00

(54) **Display of a diagnostic device**

(30) Priority: 16.02.2009 US 152823 P
(71) Applicant: Roche Diagnostics GmbH, 68305 Mannheim (DE); F.HOFFMANN-LA ROCHE AG, 4070 Basel (CH)
(72) Inventor: La Bastide, Sebastiaan, San Mateo CA 94402 (US); Balint, Eva, Mountain View CA 94040 (US)

(57) **Abstract**

The present invention relates to a diagnostic device (2) comprising a display (12), such as a blood glucose measuring device, which display includes a grid (14) which defines a plurality of spaces (16). In use, a sample of body fluid, such as blood, is obtained and the device determines a diagnostic concentration in the sample. Once the diagnostic concentration is determined, one of the spaces displays an indicia associated with the result of the diagnostic test and a character associated with the successful completion of the diagnostic test. The display also includes a score area which displays another indicia relating to the level of success of a patient's diagnostic testing regimen.

## Description

The present disclosure relates to a display of a diagnostic device, such as a blood glucose meter, and in particular, a game display and method of using the game display to encourage a patient to follow a testing regimen established by a doctor or health care provider.

Diabetes is a serious chronic disease in which the human body fails to produce or properly use insulin. According to the American Diabetes Association (ADA), there are 23.6 million children and adults in the United States, or 7.8% of the nation's population, who have diabetes. In general, there are three main types of diabetes, Type 1 diabetes, Type 2 diabetes, and Gestational diabetes. Type 1 diabetes results from the body's failure to produce insulin. The ADA estimates that of those diagnosed with diabetes, about 5-10% are diagnosed with Type 1 diabetes. Type 2 diabetes, which according to the ADA is what most Americans are diagnosed with, results from insulin resistance. Pregnant women who have never had diabetes before but who have high blood sugar (glucose) levels during pregnancy are said to have gestational diabetes. According to the ADA, gestational diabetes affects about 4% of all pregnant women - about 135,000 cases of gestational diabetes in the United States each year.

When an individual is diagnosed with diabetes, the individual must begin self-monitoring his or her blood sugar to prevent negative consequences of the disease. Monitoring blood glucose concentration becomes an essential part of the daily routine for diabetics. For example, the ADA recommends that most patients with Type 1 diabetes test glucose three or more times per day. Pregnant women taking insulin for gestational diabetes should test two times per day. ADA does not specify how often people with Type 2 diabetes should test their glucose, but testing often helps control insulin resistance. Measurement of blood glucose concentration essentially requires two procedural steps. First, a blood sample is generally produced by pricking the skin of the patient with the aid of a lancet needle. Second, once the blood sample is obtained, the sample is then analyzed for blood glucose concentration. Typically, the test strip is inserted into a glucose meter and the blood sample is then deposited onto the test strip for analysis.

The U.S. Food and Drug Administration notes that individuals with diabetes are more likely to stay healthy when they control their blood sugar through regular testing. See www.fda.gov/diabetes. However, according to Bio-Medicine and other sources, doctors find that many adults and children with diabetes fail to follow a test regimen that requires them to regularly test their blood sugar because it is inconvenient or provides discomfort. Many children, for example, choose not to regularly test their blood sugar because they encounter finger soreness, testing is inconvenient (particularly at school), and some children fear needles. Children who are old enough to attend school must deal with emotional and social issues as well. In a Guide for School Personnel, the U.S. Department of Health and Human Services (DHHS) notes that children do not want to be singled out or made to feel different from their peers. However, monitoring blood glucose can often set children with diabetes apart and make them feel angry or resentful about their disease. Such anger or resentment can lead to depression, according to DHHS, or complete disregard to regularly testing their blood sugar.

As would be expected, research confirms that those who perform fewer blood glucose tests have more hospitalizations and more doctor visits than those who test more often. According to DHHS, if diabetes is not treated, it can affect blood vessels, eyes, kidneys, nerves, gums, and teeth, and it is the leading cause of adult blindness, lower limb amputations, and kidney failure. People with diabetes also have an increased risk of heart disease and stroke. Some of these problems can occur in teens and young adults who develop diabetes during childhood.

Fortunately, research also shows that these problems can be greatly reduced or delayed by keeping blood glucose levels near normal. In one particular study, children's involvement in a health issue increases by playing video games which demonstrate positive outcomes that come from good prevention and self-care. See Lieberman, Debra A., "Health Education Video Games for Children and Adolescents: Theory, Design, and Research Findings", paper presented at the Annual Meeting of the International Communication Association, Jerusalem, Israel, 1998.

What is needed is an effective diagnostic device and method that encourages and provides incentives to diabetics to monitor their blood glucose concentration and consistently follow a predetermined testing regimen.

The present disclosure provides a diagnostic device comprising a display for use with the diagnostic device, such as, for example, a blood glucose measuring device, that includes a grid which defines a plurality of spaces. In one embodiment, a sample of blood is obtained and the device determines the blood glucose concentration in the sample. Once the blood glucose concentration is determined, one of the spaces displays both a first indicia associated with the result of the blood glucose test and a character associated with the successful completion of the blood glucose test. The display also includes a score area which displays a second indicia relating to the level of success of a patient's glucose testing regimen. Such display is hereinafter also sometimes referred to as "game display".

In one embodiment thereof, a blood glucose measuring device is provided for monitoring blood glucose concentration while also creating game-like incentives. The device includes a display that displays a grid defining a plurality of spaces, such that after a blood glucose test, the blood glucose concentration is displayed and one of the spaces displays a color or character. The color or character can be associated with a result of the blood glucose test and/or the successful completion of the blood glucose test. These embodiments are advantageous because, after each blood glucose test, immediate test results and feedback are provided to the user in an aesthetically-pleasing and entertaining manner.

In an advantageous embodiment, after a blood glucose test is completed and the blood glucose concentration is determined by the device, a base point value is awarded. The base point value is based on a percentage of scheduled blood glucose tests that are completed, and the base point value can increase when a user completes two consecutively scheduled tests (e.g., pre-prandial and post-prandial tests). One advantage associated with this dynamic scoring concept is it encourages a user with diabetes to consistently measure their blood glucose and follow a personalized testing regimen assigned by a doctor or health care provider. Also, as additional blood glucose tests are completed, the score area updates and displays the total number of points awarded to the user. Once each scheduled blood glucose test is completed over a specific time period, one or more bonus points are awarded and the score area will update and display the total number of points. For this reason, in order to maximize the number of points received over a specified time period, the user is encouraged to consistently monitor their blood glucose concentration and follow the established testing regimen.

Advantageously, the present disclosure provides an incentive-based and entertaining means for people diagnosed with diabetes to track their blood glucose concentration and consistently follow a testing regimen.

While a preferred embodiment of the diagnostic device according to the present invention is a handheld blood glucose measurement device, alternative embodiments comprise computers such as personal computers or laptops etc., which are able to receive measurement data from a separate device like a handheld blood glucose measurement device via wired or wireless data transmission.

The above-mentioned aspects of the present disclosure and the manner of obtaining them will become more apparent and the disclosure itself will be better understood by reference to the following description of the embodiments, taken in conjunction with the accompanying drawings, wherein:

FIG. 1 is a perspective view of a diagnostic device having a display in accordance with one embodiment of the present disclosure;

FIG. 2 is a flowchart in accordance with another embodiment of the present disclosure;

FIG. 3 is a schematic view of an embodiment of a game display of a diagnostic device;

FIG. 4 is a schematic view of a different embodiment of a game display of a diagnostic device; and

FIGS. 5-21 are schematic views of an exemplary embodiment of a game display of a diagnostic device.

Corresponding reference numerals are used to indicate corresponding parts throughout the several views.

The embodiments of the present disclosure described below are not intended to be exhaustive or to limit the present disclosure to the precise forms disclosed in the following detailed description. Rather, the embodiments are chosen and described so that others skilled in the art may appreciate and understand the principles and practices of the present disclosure.

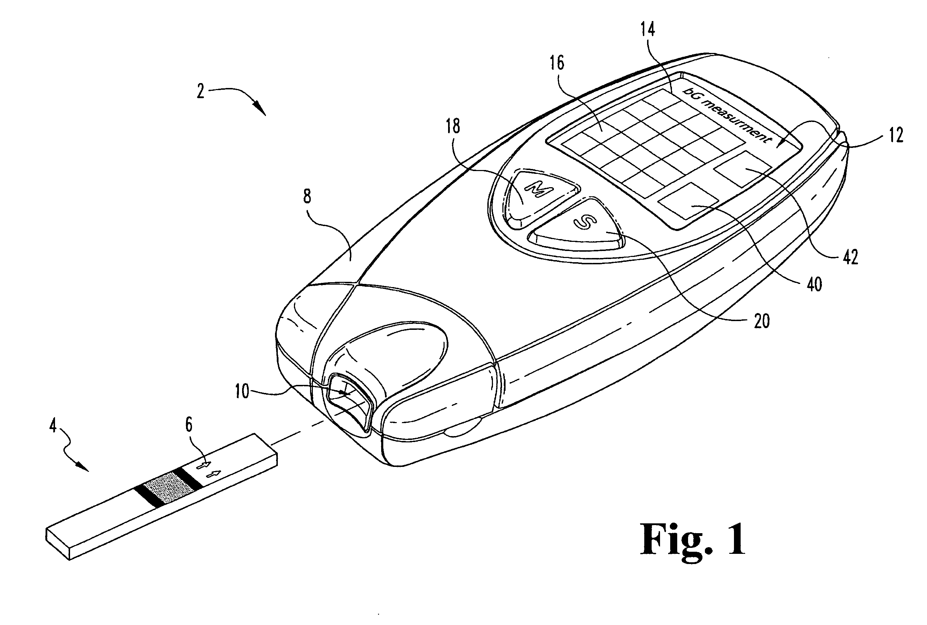

An exemplary embodiment of a diagnostic device incorporating a game display and game software is shown in FIG. 1. The diagnostic device 2 includes a housing 8 having an opening 10 and a screen display 12. The opening 10 is positioned at one end of the device 2 for receiving a test sample carrier or strip 4. The carrier or strip 4 is provided with markings 6 which indicate the direction and orientation in which the carrier or strip 4 is inserted into the opening 10. In use, the carrier or strip 4 is properly oriented and inserted through the opening 10 and into the device 2. A sample of body fluid, e.g., blood, is then obtained and deposited onto the carrier or strip 4 for analysis. Although a diagnostic device having a display is depicted in Fig. 1, it should be understood that the "display" could be provided as a display on various devices, non-limiting examples of which include a personal or laptop computer. In this example, the "diagnostic device" could be provided as a hardware component of the computer or, e.g., as a separate peripheral that connects to a computer. Various other configurations are possible and contemplated by this disclosure. The terms "display" and "diagnostic device" should thus be interpreted broadly for purposes of this disclosure.

In addition to the display 12, the diagnostic device can, for example, include one or more user keys 18, 20, a wireless communication circuit configured to transmit wireless signals and to receive wireless and remotely generated signals, and a processor including a memory having instructions stored therein. The instructions can be executable by the processor to generate screen data for the display 12 and to transmit the screen data via the wireless communication circuit. Also, the instructions can be executable by the processor to control the display to display a predefined screen that is different from the screen data and to implement remotely generated and wirelessly received medical device commands.

The instructions stored in the memory may further include instructions that are executable by the processor to display an image on the screen display 12. The instructions can include software for playing an interactive game with the diagnostic device and displaying the game images on the screen display 12. An example of a medical device having a processor including a memory having instructions stored therein is described in WO 2009/005957, which is hereby incorporated by reference.

As shown in FIG. 1, the screen display 12 includes a grid 14 that defines a plurality of spaces 16. As will be described in greater detail below, the grid 14 can define one or more columns and rows. After a diagnostic test, such as, for example, a diagnostic test of blood glucose levels, lipids levels, cholesterol level or any other suitable diagnostic test, is completed, for example, the screen display 12 can display the result of the diagnostic test and indicia or images associated with the game.

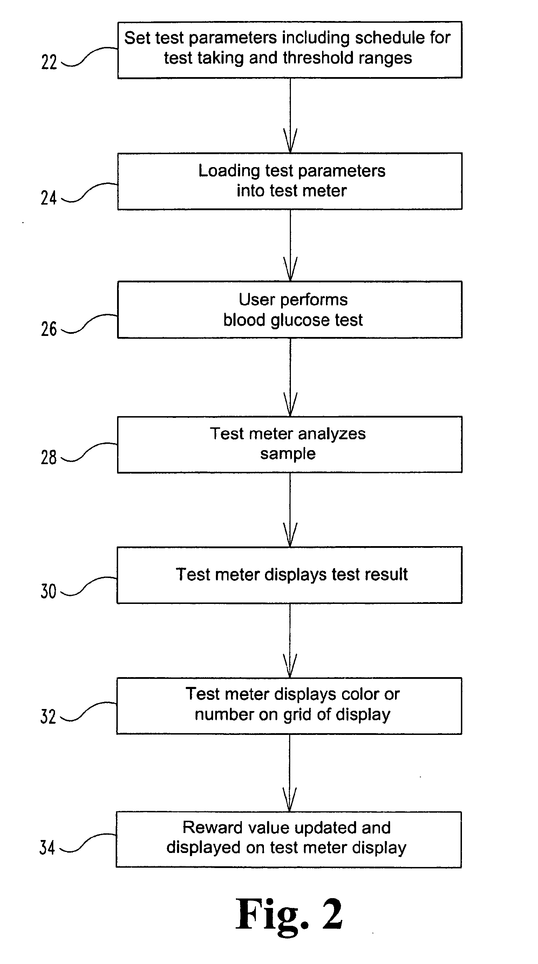

A testing regimen can be customized for each user and the instructions for carrying out the testing regimen are stored in the memory of the device. It would also be possible to upload/download the instructions from the internet or a software package. In FIG. 2, for example, a first step 22 can include establishing test parameters for each user based on the type of diabetes the user is diagnosed with and the patient's treatment plan. In one aspect, the test parameters can include a schedule which directs the user as to when one or more diagnostic tests should be completed. Advantageously, a doctor or health care provider can create the schedule and customize it for each patient. The schedule may instruct the user to complete one or more diagnostic tests during a single day, e.g., in the morning, afternoon, evening, and before bed. Alternatively, the schedule may instruct the user to complete one test before bed and then in the morning for each day during a week. One of skill in the art can appreciate that an unlimited number of test schedules can be established depending on the medical needs of a patient. The testing regimen can also be personalized to the user's individualized needs including therapy, psychology, education, and other possible needs.

In another exemplary embodiment, the doctor or health care provider can establish various diagnostic concentration ranges or limits within which a diagnostic measurement can fall. The doctor or health care provider can establish, for example, two ranges, the first range being a desirable range and the second range being a non-desirable range. For example, in the case of blood glucose measurements, the first range can be a glucose concentration under 125 mg/dL and the second range can be a glucose concentration over 125 mg/dL. For different patients, the desirable and undesirable ranges can vary, especially for hypoglycemic patients compared to hyperglycemic patients. In an alternative arrangement, the doctor or health care provider can establish three different glucose concentration ranges. In one form thereof, the first range can be up to 110 mg/dL, the second range between 110 mg/dL and 160 mg/dL, and the third range can be any blood glucose concentration over 160 mg/dL. These ranges are provided only to serve as a non-limiting example. As noted above, the doctor or health care provider establishes the ranges for each patient and customizes the ranges based on the needs of each patient. Therefore, one skilled in the art can create any number of ranges within which a blood glucose concentration may fall.

Once the test parameters have been established by the doctor or health care provider, the next step 24 in FIG. 2 is loading the test parameters into the diagnostic device, e.g., test meter. This can be done by the doctor or health care provider, for example, during an office visit by the patient. Alternatively, the test parameters can already be pre-programmed into the diagnostic device (e.g., by the manufacturer of the device) and the doctor or health care provider can select which device contains pre-programmed test parameters that closely match the needs of the patient. In another embodiment, the patient may load the test parameters into the diagnostic device. In the case of a young child, a parent or adult caretaker can load the test parameters into the device. This may also include a school nurse, for example, when a child performs one or more diagnostic measurements at school. One skilled in the art can further appreciate different methods for loading the test parameters into the diagnostic device.

Step 26 in FIG. 2 is performing a diagnostic test, such as, for example, a blood glucose test, as described above. The diagnostic device 2 then analyzes the body fluid sample (step 28) and displays the test result (step 30) in the test information area 42 of the screen display 12. With each completed diagnostic test, the grid 14 of the screen display 12 can display an indicia and/or character in a space 16 of the grid 14 (step 32). As will be described in detail below, the indicia and character can include a color, number, letter, star, checkmark, or any other symbol or marking known to one skilled in the art. Also, a reward value is updated and displayed in a score area 40 of the screen display 12 (step 34).

The doctor or health care provider can also adjust the difficulty of the game and testing regimen as the user gains more skill in testing and following the testing regimen. This can include, for example, initially testing diagnostic concentrations only in the morning, and as the user becomes more accustomed to the testing and does not miss measuring his or her diagnostic concentration, the testing regimen changes to include both pre-prandial and post-prandial testing. The testing regimen can also include food and exercise incentives such that the user is encouraged to follow a particular diet and exercise program. In contrast, if the user is uncomfortable with the testing regimen or is unsuccessful with following the testing regimen, education, training classes, and other incentives can be implemented into the user's schedule. Alarms or other reminders can also be used to help the user follow the testing regimen.

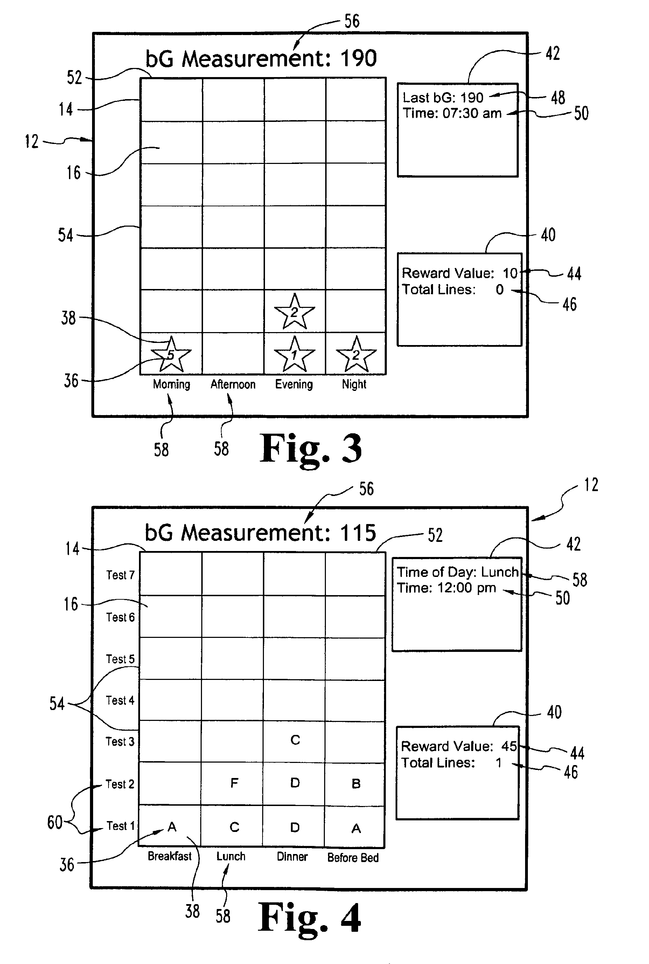

The screen display 12 and game features can be appreciated with reference to FIG. 3, which shows grid 14 having four columns 52 and seven rows or lines 54. Each column 52 can represent a time of day 58 a diagnostic test is to be completed and each row or line 54 can represent a quantity of tests to be completed over a specified time period. For instance, in FIG. 3, the time of day 58 can include morning, afternoon, evening, and night. Although in FIG. 3 the time of day 58 is displayed towards the bottom of each column 52, it is also possible to display the time of day 58 towards the top of each column 52. In other forms, it is possible to use symbols or images rather than text to display the time of day 58. One skilled in the art may appreciate alternative ways to display the time of day 58 on the screen display 12.

In FIG. 3, each of the rows or lines 54, for example, can represent a single day in a week. As described above in FIG. 2, the first step 22 of establishing a test schedule can include the testing regimen displayed in FIG. 3 in which a patient performs diagnostic tests during the morning, afternoon, evening, and night for seven days.

In FIG. 4, the testing regimen is slightly different than is illustrated with respect to FIG. 3, in that the time of day 58 includes breakfast, lunch, dinner, and before bed. The doctor or health care provider can instruct the patient to perform a diagnostic test during each of the times of day 58 at least seven times, but the patient may be given a month or longer to perform each test. In FIG. 4, each row or line 54 is indicated by a test number 60 rather than a day of the week (e.g., Sunday, Monday, Tuesday, etc.). Although in FIG. 4 the test number 60 is displayed adjacent to each row or line 54, in other embodiments of screen display 12, the test number 60 or day of the week is not displayed next to each row or line 54.

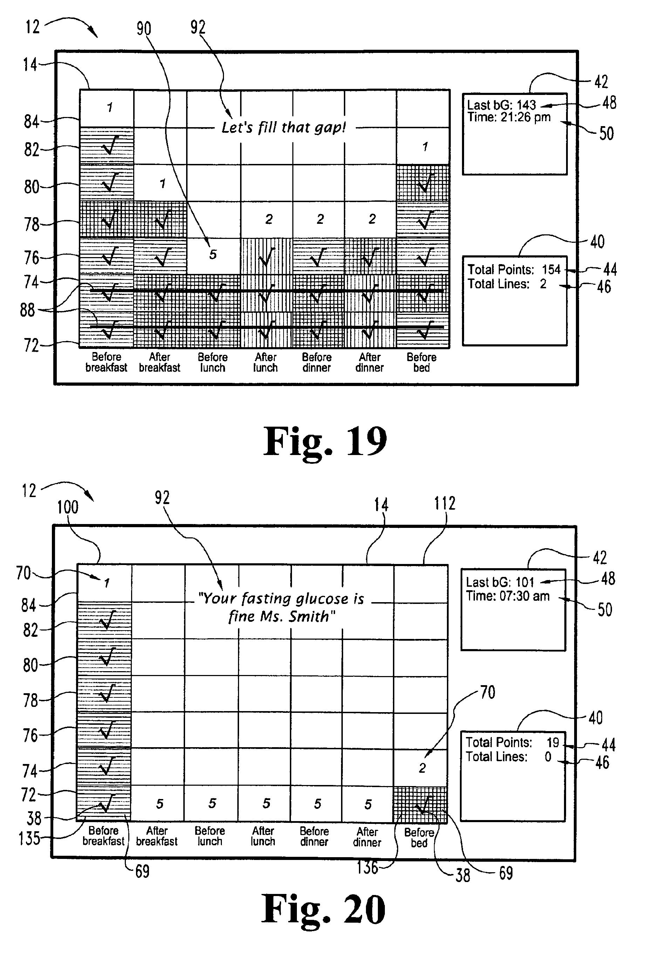

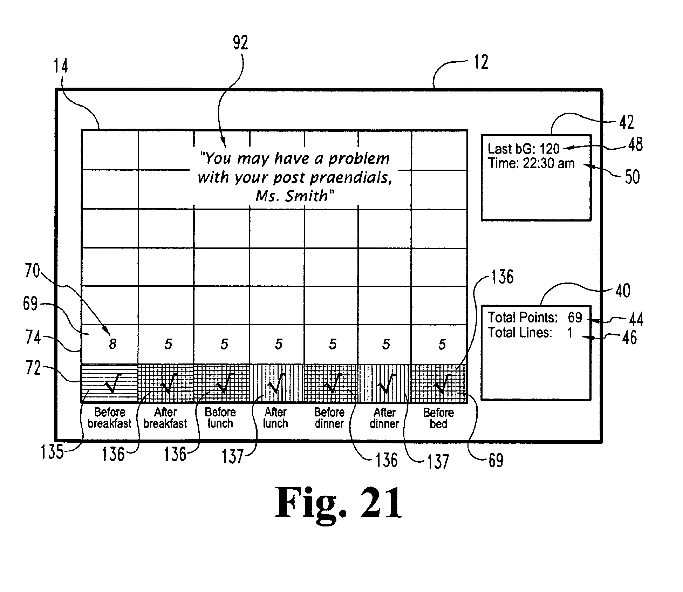

Once a patient performs a diagnostic test, the test result can be displayed in the message display area 56 of the screen display 12. While in FIG. 3 the message display area 56 is shown near the top of the display 12, the message display area 56 can also be disposed near either side of the grid 14, below the grid 14, and/or at a location within the grid (see FIGS. 19-21). In addition to the test result, the time of day 58, test time 50, and/or other messages can be displayed in the message display area 56. For example, a message encouraging the user about a complicated test can be displayed in the test area. Also, a message related to the test result, such as those messages displayed in FIGS. 20 and 21, can be displayed in the message display area 56. Other messages, such as those pre-programmed or communicated by a doctor, health care provider, etc., can be displayed in the message display area.

In addition to the test result, other information can be displayed on the screen display 12. In FIG. 3, for example, after a diagnostic test is completed, a space 16 in the grid displays an indicia 36 associated with the test result (shown as an arabic numeral 5) and a character 38 (shown as a star) associated with the successful completion of the diagnostic test. The character indicates whether a diagnostic test has been completed, whereas the indicia is related to the actual test result and can include a point value (e.g., game point). The indicia 36 can be a color, number, letter, or symbol. The character 38 can be a letter, number, symbol, shape, or point value.

In the display 12 shown in FIG. 3, the indicia is a number that is related to the diagnostic test result, such as, for example, measured blood glucose concentration. A five (5) represents the most desired user action, which in this case is a most preferred test result, viz., a test result that falls "in range" and a one (1) represents the least desired user action, which in this case is an "out of range" test result. In FIG. 3, for example, the user's glucose concentration was in the target range in the morning (i.e., the indicia 36 is displayed as a 5), but the user's glucose concentration was out of range in the evening (indicia 36 is displayed as a 1 and a 2) and night (indicia displayed as a 2). Also, the test was missed altogether in the afternoon (blank space). Feedback of actual results can alert the patient to potential problems with their exercising and/or eating habits, or other factors that may be affecting the measurements. In a different exemplary embodiment, the number one (1) can represent the most in range test result and number five (5) can represent the least in range test result.

In the screen display 16 of FIG. 3, the character is displayed as a star.
Accordingly, if the diagnostic concentration falls within a preferred range (e.g., an in range result) and the indicia is a five (5), a star is displayed in the space with a five (5) displayed inside the star, referred to hereinafter as a "five star." An example of a five star is shown in the first morning space of FIG. 3. While the space is shown with a single star and a number displayed inside the star, in a different exemplary embodiment, five smaller stars can be displayed in the space and this, too, would be referred to as a "five star."

In FIG. 3, the screen display 12 also includes a score area 40 and test information area 42. While both the score area 40 and test information area 42 are surrounded by a border in the screen display 12 of FIG. 3, this is merely an aesthetic-enhancing feature and is not required. The score area 40 can display different information such as a reward value 44 and total rows or lines 46, both of which will be described in more detail with reference to the game. It is also possible, as shown in FIG. 4, for the score area 40 to display a second indicia 44, such as total points. The test information area 42 can display various parameters about a diagnostic test, including the test result 48, the time the test was completed 50, and/or the time of day 58 of the completed test. Another test information area 48 may display the time of day of the next scheduled test or past test results. Other information known to one skilled in the art can be displayed in the test information area 42 of the screen display 12.

Screen display 12 also displays game information and data. In FIG. 3, for example, the score area 40 displays "10" as the reward value 44. The reward value 44 increases after each diagnostic test is completed based on the indicia 36 displayed in the space 16. For example, assume each space 16 in the grid 14 initially does not display an indicia or character, i.e., all spaces are blank or empty. After the first morning diagnostic test, the patient receives a five star. In this example, the reward value 44 increases from zero (0) to five (5). The first dinner diagnostic test is completed next and the patient receives one star and the reward value increases to six (6). Next, the patient completes the first night diagnostic test and receives a two star. The patient is rewarded two points and the reward value 44 now displays eight (8). Finally, as shown in the display 12 of FIG. 3, the patient performs a fourth test during the evening and receives a two star. As a result, the reward value 44 increases from eight (8) to ten (10). As more testing is completed, additional spaces 16 display an indicia 36 and character 38 and the reward value 44 is updated based on each completed test.

In the embodiment of the screen display 12 of FIG. 4, the indicia 36 and character 38 are the same, a single letter. As noted above, the display 12 includes a grid 14 having four columns 52 and seven rows 54. After completing a diagnostic test at breakfast, for example, the space 16 that falls in the column labeled "Breakfast" and row labeled "Test 1" displays the letter A. In this embodiment, the letters A, B, C, D, and F are used as first indicia and characters. Similar to the meaning attributed to letter grades in a school environment, the letter A can correspond to an excellent diagnostic test, whereas the letter F can represent a very poor diagnostic test. Likewise, the letter B can represent an above average test result, the letter C can represent an average test result, and the letter D can represent a below average test result. Of course, in other exemplary embodiments, the association given to each letter can be different and letters other than A, B, C, D, and F can be used in different embodiments.

Turning to the gaming aspect of the display 12 shown in FIG. 4, in one embodiment, the letter A is associated with five (5) points, B is four (4) points, C is three (3) points, D is two (2) points, and F is one (1) point. The value associated with each letter can vary for different embodiments. As described above with respect to FIG. 3, it is assumed that each space 16 in the grid of FIG. 4 initially is empty and therefore the reward value 44 is initially set at zero (0). As an example, the first completed diagnostic test is at breakfast and the space 16 associated with this particular test displays the letter A. In this embodiment, the letter A is associated with an excellent test result and the user receives five (5) points for the completed test. The reward value 44 in the score area 40 is updated from zero (0) to five (5) points. Also, by displaying the letter A in the space 16, not only does the letter represent an excellent test result, but its presence signifies that a diagnostic test has been successfully completed at breakfast. After the next diagnostic test is completed at breakfast, the space 16 in the column 52 labeled "Breakfast" and second row 54 labeled "Test 2" will display an indicia 36 and character 38. Thus, in this embodiment, the particular letter that appears in the space is associated with the degree of success of the test, whereas the mere presence of any letter in the space indicates that a test was performed.

As the user completes diagnostic tests for a first time at lunch, dinner, and before bed, the user is awarded three (3) points at lunch (corresponding to the letter C), two (2) points at dinner (corresponding to the letter D), and five (5) points before bed (corresponding to the letter A). As a result, the reward value 44 updates to fifteen (15) points. However, the user has also completed the first test at each of the times of day 58, namely, at breakfast, lunch, dinner, and before bed. In other words, each space 16 in the first row 54 labeled "Test 1" displays the indicia 36 and character 38 and therefore the first line is completed. By completing the first line, the user is awarded twenty (20) bonus points and the reward value 44 updates from fifteen (15) points to thirty-five (35) points. Also, the total lines 46 is updated from zero (0) to one (1). As the user continues to perform diagnostic tests, the same scoring system is applied to each test result. Thus, in the display 12 of FIG. 4, the reward value 44 displays forty-five (45) points and the total lines 46 continues to display that one (1) line has been completed. As with the other embodiments described above, the game and scoring system in FIG. 4 can be altered for different patients and the examples given are intended to be non-limiting. Different types of screen layouts can be used, different first indicia 36 and characters 38 can be displayed, and test schedules can vary depending on the doctor or health care provider and the patient.

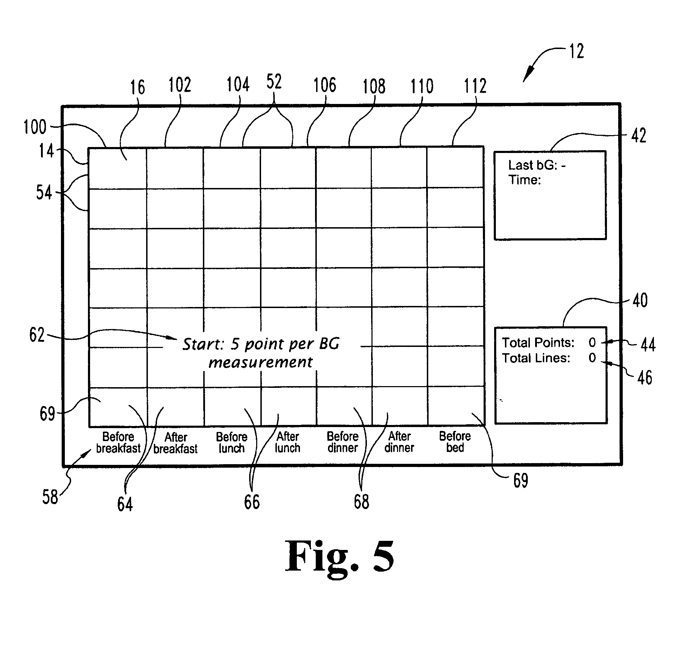

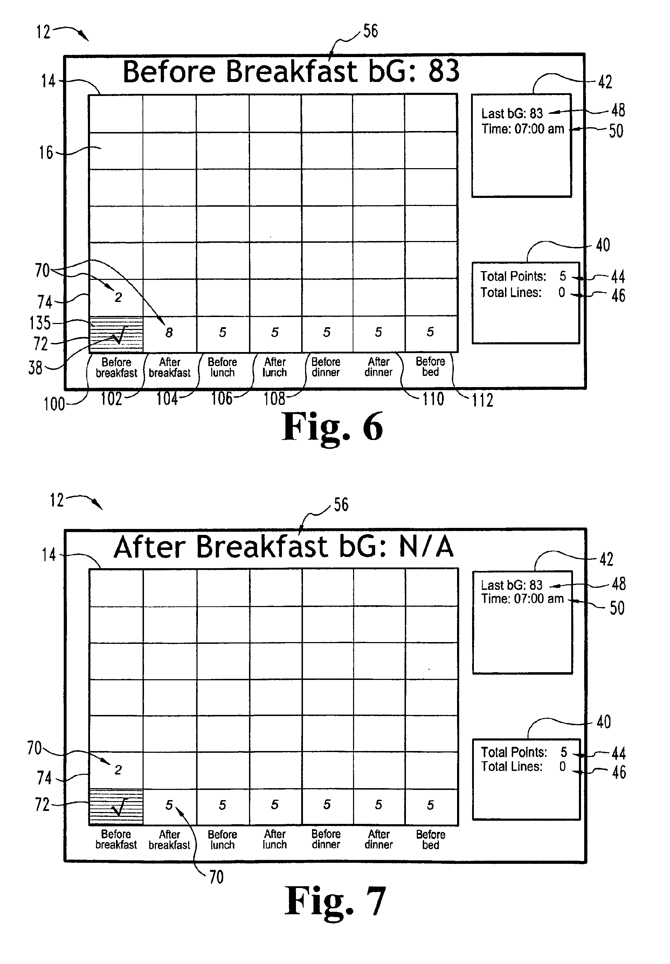

An exemplary embodiment of another screen display 12 is shown in FIG. 5. The screen display 12 includes a grid 14 with a plurality of spaces 16 defined therein. The grid 14 is divided into one or more columns 52 and rows 54, with each of the columns representing a time of day 58 in which a diagnostic test can be completed. In this embodiment, the time of day includes before breakfast, after breakfast, before lunch, after lunch, before dinner, after dinner, and before bed. Also, the columns 52 include a before breakfast column 100, after breakfast column 102, before lunch column 104, after lunch column 106, before dinner column 108, after dinner column 110, and before bed column 112. The screen display 12 can display a message 62 in the grid 14 or in a message display area 56 (see FIG. 6).

The screen display 12 also includes a score area 40 and a test information area 42. In FIG. 5, for example, the score area 40 displays a second indicia. The second indicia includes a reward value 44 and a total line value 46. The test information area 42 can display the last time a diagnostic test was performed, the result of a completed diagnostic test, past results of completed diagnostic tests, the time of day a next diagnostic test needs to be completed, or any other test information related to a diagnostic test. In FIG. 5, the test information area 42 displays the result of the most recently completed diagnostic test and the time the test was completed.

In this embodiment, a dynamic scoring system is used that provides additional incentives to encourage users to consistently monitor and test. In Table 1 below, the scoring system is summarized based on test criteria and points awarded. Points are typically awarded based on five different test criteria. For example, after the user completes a diagnostic test for the first time for a given time of day, e.g., before breakfast, the user is awarded five (5) base points. If the user repeats the same test, e.g., before breakfast the following day, without completing the other scheduled diagnostic tests, the user is awarded two (2) points. The base point value ("BPV") decreases from five (5) points to two (2) points as a result of the user not completing all scheduled tests. Similarly, if the user on the third day repeats only the test before breakfast, the user receives one (1) point. For the third and each subsequently repeated test thereafter, the BPV decreases from two (2) points to one (1) point. This will be described in more detail with reference to an example below.

The user can also be awarded bonus points for completing 1) consecutively scheduled diagnostic tests and 2) all scheduled diagnostic tests over a specified time period. For example, in FIG. 5, the times of day 58 are divided into four pairs of consecutively scheduled tests. In particular, before breakfast and after breakfast are a first pair 64, before lunch and after lunch are a second pair 66, before dinner and after dinner are a third pair 68, and before bed and before breakfast are a fourth pair 69. When one of these pairs of consecutively scheduled tests is completed, the user is awarded three (3) bonus points. The bonus points are in addition to the base point value awarded for completing the test, such that the user can actually receive a maximum of eight (8) points when one of the four pairs is satisfied. The user is also awarded twenty-five (25) bonus points when all scheduled tests over a specified time period are completed. For example, in FIG. 5, when each space 16 in the first row or line 54 displays an indicia 36 and a character 38, the user is awarded twenty-five (25) bonus points for completing the first line of tests. Likewise, as described above, the total lines 46 of the score area 40 updates and displays the total number of lines in which each space in a line displays the indicia 36 and character 38.

**TABLE 1: Scoring System**

| **Test Criteria** | **Points Awarded** |
|---|---|
| Completing a Scheduled Diagnostic Test | BPV = 5 points |
| Repeating a Previously Completed Diagnostic Test without Completing All Scheduled Diagnostic Tests (First Occurrence) | BPV = 2 points |
| Repeating a Previously Completed Diagnostic Test without Completing All Scheduled Diagnostic Tests (Each Subsequent Occurrence) | BPV = 1 point |
| Completing Consecutive Scheduled Diagnostic Tests Over a Specified Time Period | Bonus Points = 3 points |
| Completing All Scheduled Diagnostic Tests Over a Specified Time Period | Bonus Points = 25 points |

Three specific examples are provided below to further explain the features of the game described generally with reference to FIG. 5.

### Example 1

In FIG. 6, the user completes a first blood glucose test before breakfast. The glucose concentration is 83 mg/dL and this result is displayed in the message display area 56 and test information area 42 of the screen display 12. The test was completed at 7:00 A.M., as displayed in the time of testing 50 of the test information area 42. An indicia 135 and character 38 are displayed in the space 16 associated with the first "before breakfast" test. In this example, a doctor or health care provider established three blood glucose ranges within which a test result could fall, namely, a first range 135 (displayed as a first color), a second range 136 (not shown in FIG. 6), and a third range 137 (not shown in FIG. 6). The first range 135 can be an in range test result such that the blood glucose concentration falls in a preferred range set by the user's doctor or health care provider. The second or intermediate range 136 can be a test result in which the blood glucose concentration falls outside the first or preferred range 135, but does not fall within the third range (i.e., between the first and third ranges). The third range 137 can be an out of range or non-preferred test result such that the blood glucose concentration falls outside the first range 135 and second range 136 set by the doctor or health care provider.

In FIG. 6, the indicia 135 represents a blood glucose measurement that falls within the first range. Or stated differently, a blood glucose measurement of 83 mg/dL falls in a preferred or in range range and thus the space displays an in range indicia 135. Also, the character 38 is displayed as a checkmark and signifies that the first before breakfast blood glucose test is successfully completed.

As explained above, the user is also awarded five (5) points for completing the test. The reward value 44 of the score area 40 is updated and displays five (5) total points as shown. Additionally, the user is presented with the opportunity to receive bonus points for completing the first pair 64 (shown in FIG. 5) of consecutively scheduled tests, namely, "before breakfast" and "after breakfast." Each space 16 in the base row 72 displays a base point value 70. The space 16 in the base row 72 of the after breakfast column 102 displays a base point value of eight (8), which includes the five (5) points awarded for completing the test and the three (3) bonus points. Also, the space 16 in the second row 74 and before breakfast column 100 displays a base point value of two (2) because not all tests in the first row 72 have been completed. Likewise, each of the spaces 16 in the first row 72 other than the after breakfast column 102 display a base point value of five (5).

With reference to FIG. 7, the user skips the blood glucose test after breakfast. As a result, the base point value for the space 16 in the base row 72 and after breakfast column 102 resets after a specified period of time from eight (8) points shown in FIG. 6 to five (5) points shown in FIG. 7.

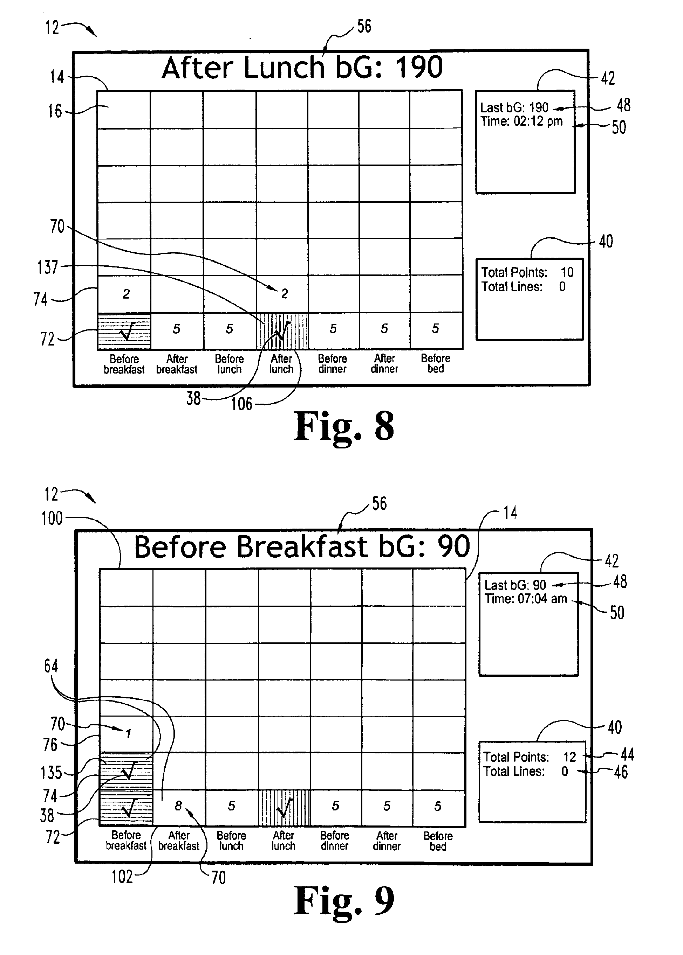

As shown in FIG. 8, the user completes a first blood glucose test after lunch. A space 16 in the base row 72 of the grid 14 in the after lunch column 106 displays an indicia 137 and character 38. The indicia 137 corresponds to a blood glucose concentration falling in the third range, i.e., corresponding to the non-preferred range and/or out of range test result as set by the doctor or health care provider. The blood glucose measurement of 190 mg/dL is displayed in the message display area 56 and test information area 42 and the user is awarded five (5) points for completing the test. The test information area 42 also displays the time of testing 50 as 2:12 P.M. and the reward value 44 increases from five (5) points to ten (10) points. Also, the space 16 in the second row 74 of the after lunch column 106 displays a base point value of two (2) points. But since tests completed after lunch and before dinner do not satisfy one of the four pairs of consecutive tests, the space 16 in the base row 72 and before dinner column 108 (see FIG. 6) displays a base point value of five (5) points.

The next blood glucose test completed by the user is again before breakfast, as illustrated in the screen display 12 of FIG. 9. The blood glucose measurement of 90 mg/dL is displayed and the space 16 in the second row 74 and before breakfast column 100 displays the indicia 135 and character 38. The test result falls in the first or preferred in range range, and the indicia 135 is thus displayed as the first color. The user is awarded two (2) points for completing the test and the reward value 44 is updated and displays twelve (12) total points. Since the user has still not completed all of the scheduled tests, i.e., the user has not completed tests after breakfast, before lunch, before dinner, after dinner, or before bed, the space 16 in a third row 76 of the before breakfast column 100 displays a base point value of only one (1) point. In this particular embodiment, the minimum number of points the user can be awarded for completing a blood glucose test is one (1) point, thereby at least always providing a small incentive to consistently monitor blood glucose. Thus, if the user continues only testing before breakfast without completing all of the scheduled tests at least once, whereby each space 16 in the base row 72 displays an indicia and character, the user is at least awarded one (1) point. However, by completing the test before breakfast, the increased point value for completing the first pair 64 of consecutively scheduled tests is available and the space 16 in the base row 72 and after breakfast column 102 thus displays a base point value of eight (8) points.

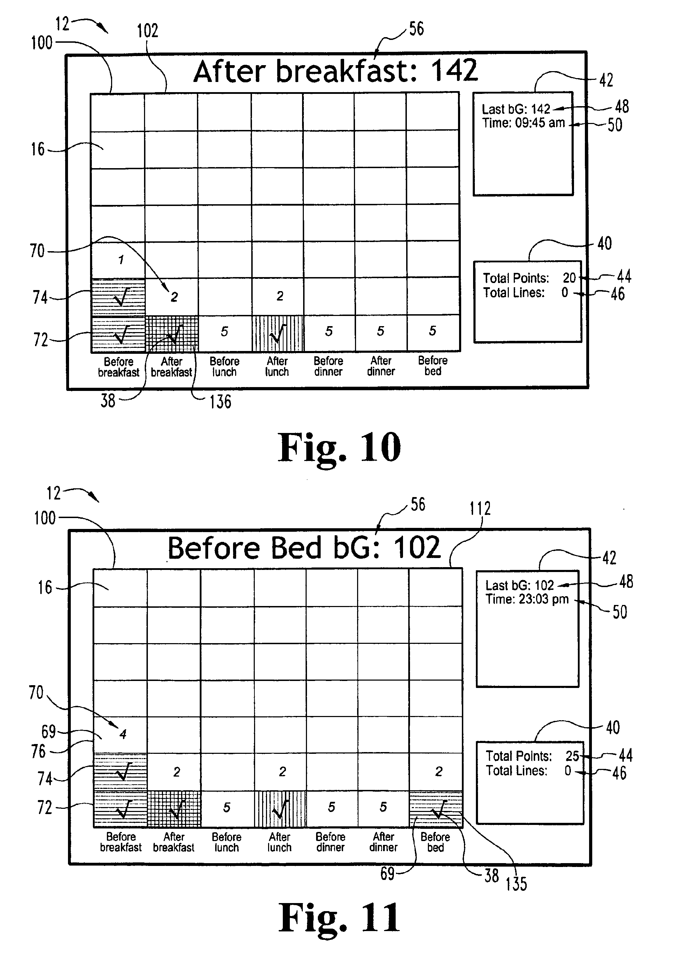

Turning to FIG. 10, the user next completes the first pair 64 of consecutively scheduled tests by performing a blood glucose measurement after breakfast. As a result, the space 16 in the base row 72 and after breakfast column 102 displays a character 38 and indicia 136 which is associated with a test result falling in the second or intermediate range, and the second color is thus displayed in the space 16. The user is awarded eight (8) points for completing the test and the reward value 44 is updated from twelve (12) points to twenty (20) points. The space 16 in the second row 74 and after breakfast column 102 displays a base point value of two (2) points, for reasons described above.

Next, in FIG. 11, the screen display 12 shows that the user completes a blood glucose test before bed and the blood glucose concentration measured 102 mg/dL. This measurement 48 is displayed in the message display area 56 and test information area 42 along with the time of testing 50. Because the concentration falls within the first or in range preferred range, the space 16 in the base row 72 and before bed column 112 displays a positive indicia 135 and character 38. The reward value 44 of the score area 40 is updated from twenty (20) points to twenty-five (25) points. The space 16 in the second row 74 and before bed column 100 displays a base point value of two (2) points for reasons described above, but more importantly, the user is enticed to perform the next blood glucose measurement before breakfast to complete the fourth pair 69. Therefore, the space 16 in the third row 76 and before breakfast column 100 displays a base point value of four (4) points, which is an increase of three (3) bonus points due to the incentive of performing consecutively scheduled tests.

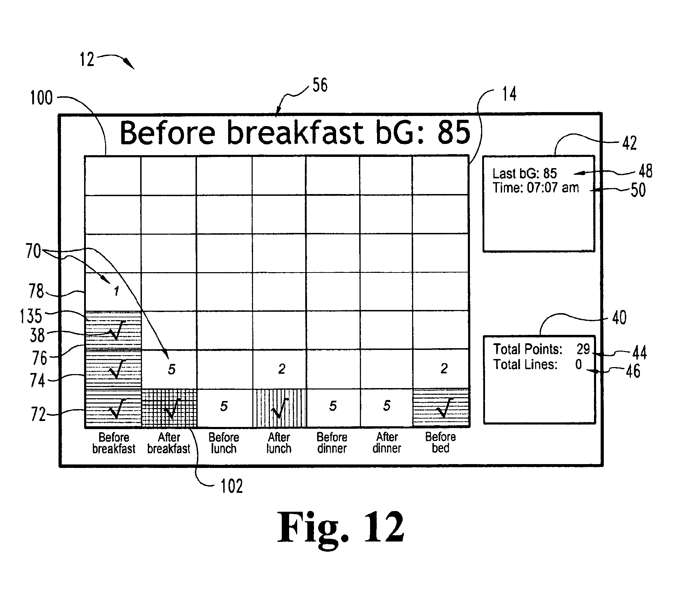

As shown in FIG. 12, the user next completes a blood glucose test before breakfast and thereby satisfies the fourth pair 69 (see FIG. 11) of consecutively scheduled tests. The space 16 in the third row 76 and before breakfast column 100 displays the indicia 135 and character 38. The glucose concentration measures 85 mg/dL and the result is displayed in the message display area 56 and test information area 42 of the screen display 12. Since the fourth pair 69 (see FIG. 11) of tests was completed, the user is awarded four (4) points and the reward value 44 updates to twenty-nine (29) total points. Additionally, testing before breakfast creates another incentive for the user to collect bonus points by completing an after breakfast test, thereby satisfying the first pair 64 (see FIG. 5) of consecutively scheduled tests. As a result, the space 16 in the second row 74 and after breakfast column 102 displays a base point value of five (5) points, which is an increase from two (2) points due to the three (3) bonus points available.

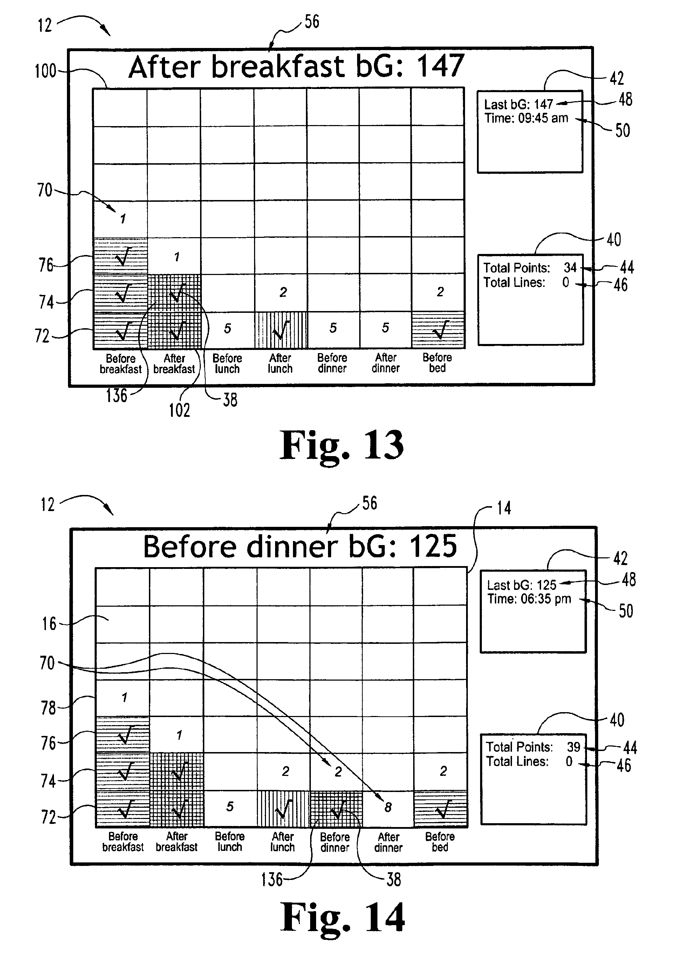

In the screen display 12 shown in FIG. 13, the user completes a blood glucose test after breakfast and therefore satisfies the first pair 64 (see FIG. 5) of consecutively scheduled tests. Consequently, the user is awarded five (5) points and the reward value 44 increases from twenty-nine (29) points to thirty-four (34) points. The space 16 in the second row 74 and after breakfast column 102 displays the indicia 136 related to the test result falling in the second or intermediate range and character 38 associated with the successful completion of the blood glucose test.

As a further illustration of how the screen display 12 provides helpful information related to the user's health to the user and doctor or health care provider, the screen display 12 shown in FIG. 13 displays indicia for blood glucose tests performed before breakfast and after breakfast. For tests performed before breakfast, the blood glucose concentration measured favorably and the indicia 135 in this column signifies preferred in range tests. This type of pattern suggests that the user's pre-prandial blood glucose concentration is satisfactory. However, after breakfast, the blood glucose concentration tends to increase and the indicia 136 in this column 102 falls within the intermediate range, possibly indicating post-prandial problems. Such a result may suggest that the user is not eating a healthy breakfast, for example. The doctor or health care provider may suggest different eating habits or other courses of action to the user, such as requesting that the user perform additional glucose measurements after breakfast.

In FIG. 14, the user next performs a blood glucose test before dinner. The glucose concentration measures 125 mg/dL, which is displayed in the message display area 56 and test information area 42. The user is awarded five (5) points for completing the test and the reward value 44 now displays thirty-nine (39) total points. The space 16 corresponding to the base row 72 and before dinner column 108 displays a character 38 and indicia 136 associated with the test result falling in the second or intermediate range. Since before dinner and after dinner tests form the third pair 68 (see FIG. 5) of consecutively scheduled tests, the space 16 in the base row 72 and after dinner column 110 displays a base point value of eight (8) points, which includes the three (3) bonus points awarded for completing the third pair 68.

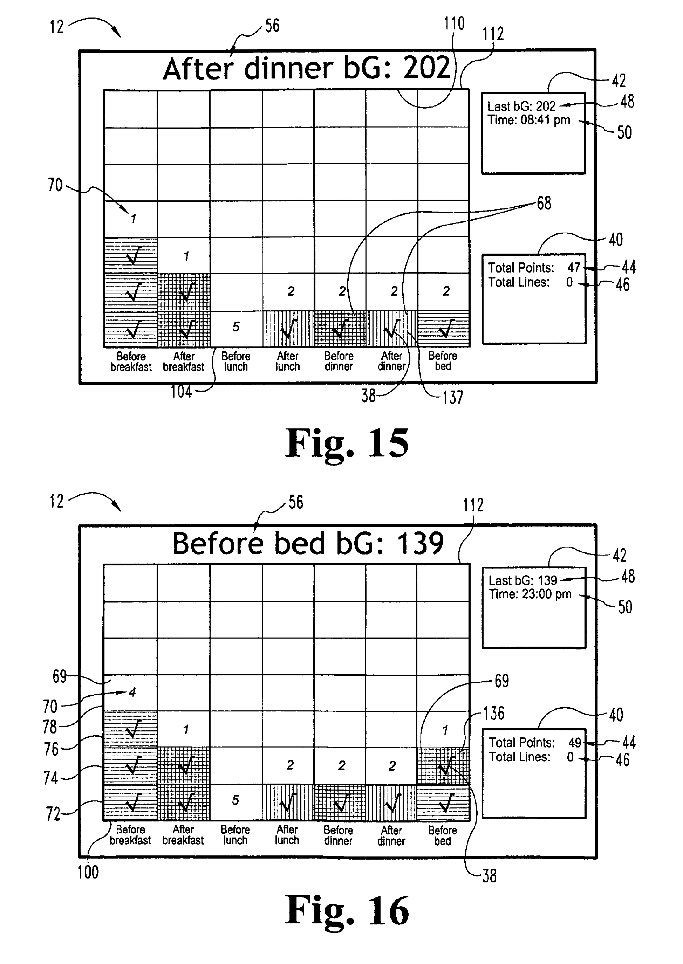

In FIG. 15, the screen display 12 illustrates the completion of a blood glucose test after dinner. The space 16 in the base row 72 and after dinner column 110 displays a character 38 and indicia 137 in which the test result falls within the third range (i.e., non-preferred out of range). Once again, the user is awarded eight (8) points for completing the scheduled test and the third pair 68. As a result, the reward value 44 increases by eight (8) points and now displays forty-seven (47) total points. Also, as shown in FIG. 15, the only remaining scheduled blood glucose test in the base row 72 not already completed is before lunch. Thus, the space 16 in the base row 72 and before lunch column 104 displays a base point value of five (5) points, whereas each of the spaces in the second row 74 and after lunch, before dinner, and after dinner columns displays a base point value of two (2) points. Finally, since blood glucose tests have been performed two or more times before breakfast and after breakfast, spaces in each of those columns display a base point value of one (1) point.

In FIG. 16, the user completes a blood glucose test before bed and the screen display 12 displays the blood glucose measurement 139 mg/dL in the message display area 56 and test information area 42. The space in the second row 74 and before bed column 112 displays a character 38 and indicia 136 associated with the intermediate range. The user is awarded two (2) points for completing the test and the reward value 44 displays forty-nine (49) total points. Additionally, by completing the test before bed, the user can accumulate three (3) bonus points by completing the fourth pair 69 (see FIG. 5) of consecutively scheduled tests if the next completed test is before breakfast.

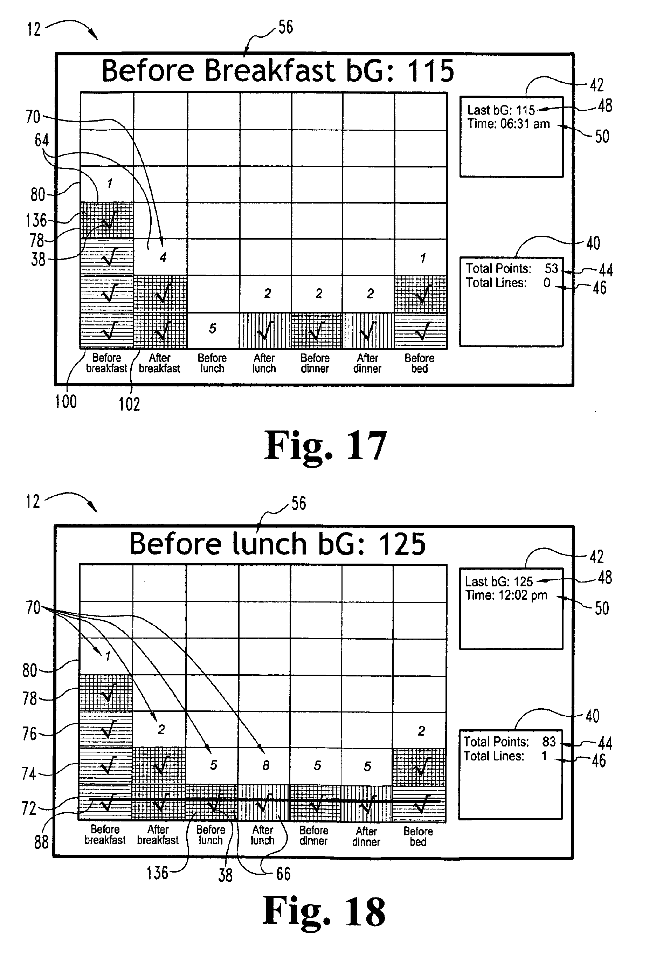

As shown in FIG. 17, the user completes the fourth pair 69 of tests by performing a blood glucose test before breakfast. The user is awarded four (4) points for completing the test and the reward value 44 displays fifty-three (53) points. Also, if the next test completed by the user is after breakfast, the first pair 64 is satisfied and the user is awarded three (3) bonus points in addition to the one (1) base point. The space 16 in the fourth row 78 and before breakfast column 100 displays a character 38 and indicia 136 representative of the blood glucose measurement falling in the second range. The space 16 in a fifth row 80 and before breakfast column 100 displays a base point value of one (1) point again.

In the screen display shown in FIG. 18, the user finally completes the last remaining scheduled blood glucose test, i.e., before lunch. The blood glucose concentration measured 125 mg/dL, which is displayed in the message display area 56 and test information area 42. The space 16 in the base row 72 and before lunch column 104 displays a character 38 associated with successfully completing the test and an indicia 136 associated with the test result falling in the second or intermediate range. The user is awarded five (5) points for completing this test for the first time. Also, the user is awarded twenty-five (25) bonus points for having completed all scheduled tests for a first time. This is shown in FIG. 18 with a complete line indicia 88 displayed in the base row 72. Thus, the score area 40 is updated such that the reward value 44 displays eighty-three (83) total points, an increase of thirty (30) points, and the total lines 46 displays one (1) completed row or line.

Once each space 16 in the base row 72 displays a character and indicia, the user is encouraged to continue performing blood glucose tests so that each space 16 in the second row 74 displays a character and indicia. To provide an incentive, each space 16 in the second row 74 and before lunch column 104, before dinner column 108, and after dinner column 110 displays a base point value of five (5) points. This is an increase from the base point value of two (2) points, as was the case in FIG. 17. Since the last test performed was before lunch, the user is further enticed to complete the next test after lunch to complete the second pair 66, and thus the space 16 in the second row 74 and after lunch column 106 displays a base point value of eight (8) points. This takes into account three (3) bonus points for completing the second pair 66 of consecutively scheduled tests. Likewise, the spaces 16 in the third row 76 and after breakfast column 102 and before bed column 112 display a base point value of two (2) points. Once again, for reasons described above with reference to Table 1, the space 16 in the fifth row 80 and before breakfast column 100 continues to display a base point value of one (1) point.

The screen display 12 shown in FIG. 19 is after the user completes additional blood glucose tests. The user, for example, has completed six tests before breakfast. The space 16 in the fifth row 80 displays a character 38 and indicia 136 associated with an intermediate test result, and likewise, the space 16 in a sixth row 82 displays a character 38 and first indicia 136 as well. The space 16 in a seventh row 84 and before breakfast column 100 displays a base point value of one (1) point, for reasons described above. Also, the user has completed two rows of scheduled blood glucose tests, i.e., the base row 72 and second row 74. The total lines value 46 of the score area 40 displays that two (2) lines have been completed and the reward value 44 displays one-hundred fifty-four (154) total points have been awarded to the user. The screen display 12 can also display a message 92 in the grid 14 or, alternatively, in the message display area 56. The message 92 can encourage the user to perform a blood glucose test such that an empty space or gap 90 exists within the grid 14.
The message 92 can also point out encouraging or concerning signs related to completed blood glucose tests, such as those displayed in FIGS. 20 and 21.

### Example 2

The screen display 12 shown in FIG. 20 represents another example of the game and scoring system described in Table 1. In FIG. 20, the user is instructed by her doctor to perform blood glucose tests for one week, and particularly, once before bed at the beginning of the week and then before breakfast each day thereafter. The doctor has established three ranges similar to those used in Example 1 above, such that indicia displayed in the grid 14 are associated with blood glucose concentrations falling in a preferred in range target range 135, an intermediate range 136, or an out of range undesired range 137.

According to the screen display 12, the space 16 in the base row 72 and before bed column 112 displays a character 38 and indicia 136 associated with an intermediate test result. However, each of the completed blood glucose tests before breakfast has been in the in range preferred range. Each space 16 in the before breakfast column 100 and base row 72, second row 74, third row 76, fourth row 78, fifth row 80, and sixth row 82 displays an indicia 135 associated with a preferred test result. Once again, a character 38 in the form of a checkmark is displayed in each space 16 and is associated with a successfully completed blood glucose test. The screen display 12 also displays the blood glucose measurement 48 and test time 50 in the test information area 42. The screen display 12 also displays the score area 40 with the reward value 44 and total lines value 46 displayed therein.

As described above, the user receives five (5) points for completing the initial before bed and before breakfast tests, but in this example, because the user completed the before bed test first, the user also receives three (3) bonus points for completing the fourth pair 69 of consecutively scheduled tests. The user then receives two (2) points for completing the second before breakfast test and one (1) point for each subsequently completed before breakfast test. Based on the completed tests shown in FIG. 20, the reward value 44 displays nineteen (19) total points.

As noted above, the blood glucose measurements before breakfast measured in the in range preferred range. Therefore, such results may indicate that the user's fasting glucose, i.e., overnight fasting, is satisfactory. A message 92 can be displayed in the screen display 12 indicating the same.

### Example 3

In the screen display 12 illustrated in FIG. 21, the user has completed each scheduled blood glucose test over a specific period of time, e.g., a single day. By doing so, the user is awarded five (5) points for each completed test and three (3) bonus points for completing the first pair 64, second pair 66, and third pair 68 of consecutively scheduled tests (see FIG. 5 for each pair). Accordingly, the reward value 44 of the score area 40 displays sixty (60) total points awarded and the total lines value 46 displays one (1) completed line, namely, the base row 72. Other similar information is displayed, including the blood glucose measurement 48 and testing time 50 in the test information area 42. Additionally, a message 92 is displayed in the screen display 12 alerting the user to a potential problem with the user's post-prandial blood glucose concentration. As shown in FIG. 21, each space 16 in the base row 72 and after breakfast column 102, after lunch column 106, and after dinner column 110 displays an indicia associated with a blood glucose concentration falling in either the second (e.g., intermediate) range 136 or third (e.g., non-preferred) range 137. Such information can be useful to both the user and doctor or health care provider in changing eating habits and the like. Other messages can be displayed in the screen display 12 as well, and one skilled in the art can appreciate that the content of those messages can vary based on the user and doctor or health care provider.

While exemplary embodiments incorporating the principles of the present disclosure have been disclosed hereinabove, the present disclosure is not limited to the disclosed embodiments. Instead, this application is intended to cover any variations, uses, or adaptations of the disclosure using its general principles. Further, this application is intended to cover such departures from the present disclosure as come within known or customary practice in the art to which this disclosure pertains and which fall within the limits of the appended claims.

The following is a list of preferred embodiments of the present invention:
1. A display, in particular a game display for use with a diagnostic device, or a device comprising the display, the display comprising:
   a grid defining a plurality of spaces, each space being configured to display a first indicia associated with a result of a diagnostic test and a character associated with successful completion of the diagnostic test; and
   a score area which displays a second indicia associated with the level of success of a patient's diagnostic testing regimen.
2. The device or display of embodiment 1, wherein the diagnostic device is a blood glucose meter.
3. The device or display of embodiment 1, wherein the diagnostic test is a blood glucose test.
4. The device or display of embodiment 1, wherein the first indicia comprises a color.
5. The device or display of embodiment 4, wherein the color fills the space.
6. The device or display of embodiment 1, wherein the character comprises a number, an Arabic numeral, a letter or combinations thereof.
7. The device or display of embodiment 1, wherein the symbol comprises a checkmark, a star, or combinations thereof.
8. The device or display of embodiment 1, further comprising a test result area which displays the result of the completed diagnostic test.
9. The device or display of embodiment 1, wherein the plurality of spaces are divided into columns and rows, each column associated with a different time of day of the patient's diagnostic testing regimen.
10. The device or display of embodiment 9, wherein the time of day comprises before breakfast, after breakfast, before lunch, after lunch, before dinner, after dinner, and before bed.
11. The device or display of embodiment 1, wherein the second indicia comprises a point value, a letter or combinations thereof.
12. The device or display of embodiment 1, wherein the score area displays a number associated with completed diagnostic tests.
13. A method of playing a game with a diagnostic device to encourage a patient to consistently follow a diagnostic testing regimen, the device comprising a display that includes a grid defining a plurality of spaces, the method comprising:
   (a) obtaining a sample of body fluid;
   (b) using the device to determine a diagnostic concentration in the sample;
   (c) displaying the diagnostic concentration on the display;
   (d) displaying in one of the spaces a color or character;
   (e) updating and displaying a score area of the display; and
   (f) repeating steps (a)-(e) several times and thereby completing several diagnostic tests.
14. The method of embodiment 13, further comprising awarding a base point value for each completed diagnostic test.
15. The method of embodiment 14, wherein the base point value is based on a percentage of scheduled diagnostic tests that are completed.
16. The method of embodiment 14, further comprising awarding a higher base point value for completing two consecutively scheduled tests.
17. The method of embodiment 16, wherein the two consecutively scheduled tests comprise pre-prandial and post-prandial.
18. The method of embodiment 16, wherein the two consecutively scheduled tests comprise before breakfast and after breakfast, before lunch and after lunch, or before dinner and after dinner, or before bed on a first day and before breakfast on a second day, the second day immediately following the first day.
19. The method of embodiment 14, further comprising awarding one or more bonus points for completing each scheduled diagnostic test over a specified time period.
20. The method of embodiment 19, wherein the specified time period is one day.
21. The method of embodiment 14, further comprising decreasing the base point value for missing a scheduled diagnostic test.
22. The method of embodiment 14, wherein the awarding step takes place before the updating step.
23. The method of embodiment 13, wherein the diagnostic concentration is displayed in the score area.
24. The method of embodiment 13, further comprising displaying the time of day a diagnostic test is completed.
25. The method of embodiment 13, wherein the displaying in one of the spaces of a color or character comprises displaying the color green, yellow, or red; a letter or number; a point value; a checkmark or combinations thereof.
26. The method of embodiment 13, further comprising changing the diagnostic testing regimen to match a skill level of the patient.

## Claims

1. A diagnostics device comprising a display, in particular a game display for use with the diagnostic device, the display comprising:
a grid defining a plurality of spaces, each space being configured to display a first indicia associated with a result of a diagnostic test and a character associated with successful completion of the diagnostic test; and
a score area which displays a second indicia associated with the level of success of a patient's diagnostic testing regimen.

2. The device of claim 1, in which the display further comprises a test result area which displays the result of the completed diagnostic test.

3. The device of claim 1 or 2, wherein the plurality of spaces are divided into columns and rows, each column associated with a different time of day of the patient's diagnostic testing regimen.

4. The device of claim 3, wherein the time of day comprises before breakfast, after breakfast, before lunch, after lunch, before dinner, after dinner, and before bed.

5. The device of any of the preceding claims, wherein the score area displays a number associated with completed diagnostic tests.

6. A method of displaying information with a diagnostic device, the device comprising a display that includes a grid defining a plurality of spaces, the method comprising:
(a) using the device to determine a diagnostic concentration in a sample of body fluid;
(b) displaying the diagnostic concentration on the display;
(c) displaying in one of the spaces a color or character;
(d) updating and displaying a score area of the display; and
(e) repeating steps (a)-(d) several times and thereby completing several diagnostic tests.

7. The method of claim 6, further comprising awarding a base point value for each completed diagnostic test.

8. The method of claim 7, wherein the base point value is based on a percentage of scheduled diagnostic tests that are completed.

9. The method of claim 7 or 8, further comprising awarding a higher base point value for completing two consecutively scheduled tests.

10. The method of claim 9, wherein the two consecutively scheduled tests comprise pre-prandial and post-prandial tests.

11. The method of claim 9, wherein the two consecutively scheduled tests comprise tests taken before breakfast and after breakfast, or before lunch and after lunch, or before dinner and after dinner, or before bed on a first day and before breakfast on a second day, the second day immediately following the first day.

12. The method of claim 7, further comprising awarding one or more bonus points for completing each scheduled diagnostic test over a specified time period and/or decreasing the base point value for missing a scheduled diagnostic test.

13. The method of claim 12, wherein the specified time period is one day.

14. The method of claim 7, wherein the awarding step takes place before the updating step.

15. The method of claim 6, wherein the diagnostic concentration is displayed in the score area.

16. The method of claim 6, further comprising displaying the time of day a diagnostic test is completed.

17. The method of claim 6, further comprising changing the diagnostic testing regimen to match a skill level of the patient.
